# FASCICULE DE BREVET EUROPEEN

(11) **EP 4 157 425 B1**
(45) Date de publication et mention de la délivrance du brevet: **07.08.2024**
(21) Numéro de dépôt: 21734427.4
(22) Date de dépôt: 01.06.2021
(51) Int. Cl.: A61M 37/00

(54) **APPLICATEUR A MICRO-AIGUILLES**
MIKRONADELAPPLIKATOR
MICRONEEDLE APPLICATOR

(30) Priorité: 02.06.2020 FR 2005785
(43) Date de publication de la demande: 05.04.2023
(73) Titulaire: APTAR France SAS, 27110 Le Neubourg (FR)
(72) Inventeur: DAVIOT, Stéphane, 27110 GRAVERON SEMERVILLE (FR); MOREAU, Francis, 76300 SOTTEVILLE LES ROUEN (FR)
(74) Mandataire: CAPRI
(86) Numéro de dépôt international: PCT/FR2021/050990
(87) Numéro de publication internationale: WO 2021/245348

(56) Documents cités:
- CN-U- 204 293 680
- KR-B1- 101 492 732
- KR-B1- 101 492 732
- US-A1- 2015 344 834
- US-A1- 2017 354 810

## Description

La présente invention concerne un applicateur à micro-aiguilles pour appliquer un produit fluide sur la peau et le faire pénétrer dans la couche supérieure de l'épiderme. Le domaine d'application de l'invention est celui de la cosmétique, et non pas celui du tatouage. Le but est de renforcer l'efficacité d'un traitement cosmétique dans la peau, et non pas de colorer la peau.

De manière conventionnelle, ce type d'applicateur cosmétique comprend une face d'application pourvue d'au moins une sortie de produit fluide et de plusieurs micro-aiguilles. Un moteur, souvent électrique, est utilisé pour faire vibrer les micro-aiguilles, seules ou avec la face d'application. Un réservoir de produit fluide est relié à la sortie de produit fluide. Le réservoir peut être intégré ou non à l'applicateur. Lorsqu'il est intégré, il se pose alors la question de son remplissage ou de son remplacement. L'applicateur peut être démontable ou comprendre une fenêtre pour accéder au réservoir.

D'autre part, un organe d'actionnement est aussi prévu pour acheminer le produit fluide du réservoir de produit fluide à la sortie de produit fluide. Cet organe d'actionnement est souvent celui qui commande aussi le moteur, qui cumule alors une double fonction, à savoir de faire vibrer les micro-aiguilles et d'acheminer le produit fluide du réservoir à la face d'application.

KR 101 492 732 B1 et CN 204 293 680 U décrivent des systèmes amovibles d'applicateur à micro-aiguilles avec moteur pour délivrer une substance.

Le but de la présente invention est de proposer un applicateur très simple, aussi bien en terme de conception et d'utilisation, avec une distribution de produit fluide simple et intuitive. L'invention est définie dans la revendication 1. D'autres aspects sont définis dans les revendications dépendantes 2 à 15.

Pour atteindre ce but, la présente invention prévoit que l'applicateur comprenne deux modules distincts raccordés axialement l'un à l'autre de manière amovible, à savoir :
- un premier module abritant le moteur et des accessoires pour faire fonctionner le moteur, et
- un second module abritant le réservoir de produit fluide et formant la face d'application et l'organe d'actionnement, l'organe d'actionnement manuel étant accessible et manipulable manuellement, lorsque les deux modules sont connectés l'un à l'autre.

La distribution de produit fluide est donc gérée manuellement par l'utilisateur qui agit à l'aide d'un doigt sur l'organe d'actionnement. On comprend alors que le second module est techniquement très simple, puisqu'il ne comprend aucun composant électrique ou électronique. Il constitue une cartouche remplaçable peu coûteuse, que l'utilisateur peut facilement séparer et connecter au premier module, qui lui, intègre le moteur, son alimentation et sa commande, et constitue de ce fait un module complexe et coûteux. Cette séparation de l'applicateur en deux modules distincts, l'un très simple et bon marché et l'autre complexe et coûteux, est rendue possible par l'actionnement manuel de la distribution du produit cosmétique par l'utilisateur.

Avantageusement, le réservoir de produit fluide est à volume variable, le déplacement de l'organe d'actionnement engendrant une diminution de volume du réservoir de produit fluide, de sorte qu'une partie de son contenu est refoulée vers la sortie de produit fluide. Ainsi, l'organe d'actionnement agit directement sur le réservoir sans organe intermédiaire, comme une pompe ou un moteur.

L'organe d'actionnement peut être un poussoir latéral, qui peut être choisi parmi :
- un ou plusieurs poussoir(s) à déplacement transversal par rapport à l'axe longitudinal X,
- un poussoir coulissant parallèlement à l'axe longitudinal X, et
- un poussoir rotatif qui tourne autour de l'axe longitudinal X.

Avantageusement, le second module peut comprendre une extrémité de connexion pourvue de moyens de couplage au premier module pour transmettre les vibrations générées par le moteur et des moyens de transmission pour transmettre aux micro-aiguilles les vibrations reçues par les moyens de couplage. Selon une forme de réalisation particulière, les moyens de transmission peuvent être formés par le réservoir. Dans certains cas, le réservoir peut être débrayable des moyens de couplage entre une position de repos débrayée et une position active embrayée, l'organe d'actionnement amenant le réservoir en position embrayée. Le réservoir remplit ainsi une autre fonction, à savoir de transmettre la force générée par le moteur.

Selon un autre mode de réalisation, les moyens de transmission peuvent comprendre une tige de transmission, le réservoir s'étendant à côté de la tige de transmission. En variante, les moyens de transmission peuvent comprendre une plaque de transmission, le réservoir de produit fluide comprenant une poche souple reposant sur la plaque de transmission, l'organe d'actionnement écrasant la poche souple sur la plaque de transmission. Les moyens de transmission peuvent donc participer à la distribution du produit cosmétique.

Selon une autre caractéristique, le réservoir, l'organe d'actionnement et la sortie de produit fluide peuvent être réalisés de manière monobloc, par exemple avec un matériau élastomérique.

L'applicateur peut présenter une configuration générale de stylo, adapté à être saisi entre le pouce et le majeur avec l'organe d'actionnement actionnable au moyen de l'index, le premier module, et avantageusement aussi le second module, pouvant présenter une section transversale sensiblement triangulaire.

Selon un mode de réalisation pratique, le second module peut être raccordé de manière amovible au premier module par une connexion combinant un mouvement axial et un mouvement rotatif.

Selon un autre aspect, la face d'application peut comprendre une platine dotée de micro-aiguille et traversée par la sortie de produit fluide. La sortie de produit fluide peut également se faire en périphérie de la face d'application.

Avantageusement, l'applicateur peut en outre comprendre des moyens d'ajustement de la profondeur de pénétration des micro-aiguilles, agissant avantageusement sur la position axiale du moteur dans le premier module. En effet, il est parfois utile de faire pénétrer plus ou moins les micro-aiguilles en fonction du produit cosmétique, du traitement souhaité ou de la qualité et la nature de la peau.

L'applicateur peut également comprendre des moyens de commande du moteur, qui le désactivent automatiquement lorsque les deux modules ne sont pas connectés. Il s'agit d'une sécurité d'utilisation.

L'effet technique de l'invention réside dans le fait de constituer un module remplaçable et donc bon marché, à la manière d'une cartouche, n'intégrant aucun organe actif (électrique) : la simplicité de ce module résulte de la mise en oeuvre d'un organe d'actionnement manuel, qui agit de préférence directement sur le volume du réservoir.

L'invention sera maintenant plus amplement décrite en référence aux dessins joints qui donnent, à titre d'exemples non limitatifs, plusieurs modes de réalisation de l'invention.

Sur les figures :
La figure 1a est une vue en coupe transversale verticale à travers un applicateur de l'invention à l'état démonté,
La figure 1b est vue agrandie d'un détail de la figure 1a,
La figure 2 est une vue perspective de l'applicateur de la figure 1,
La figures 3 est une vue en perspective d'une partie du second module de l'applicateur des figures 1 et 2,
La figures 4 est une vue en perspective d'une partie du premier module de l'applicateur des figures 1 et 2,
Les figures 5 à 9 sont des vues schématiques en coupe transversale à travers des seconds modules selon six modes de réalisation différents de l'invention.

L'applicateur de l'invention est purement cosmétique, voire dermatologique, à l'exclusion du tatouage. Il associe deux moyens de traitement, à savoir la distribution d'un produit cosmétique, qui peut être une crème, une pommade, une lotion, un sérum, etc., et la perforation de l'épiderme, sans toucher le derme, au moyen de micro-aiguilles. Selon la nature du produit cosmétique et le résultat recherché, le produit cosmétique est appliqué avant, en même temps ou après la micro-perforation. L'applicateur de l'invention est plutôt à usage domestique, en ce sens que l'utilisateur de l'applicateur va l'utiliser sur lui-même. Il peut toutefois s'utiliser de manière professionnelle.

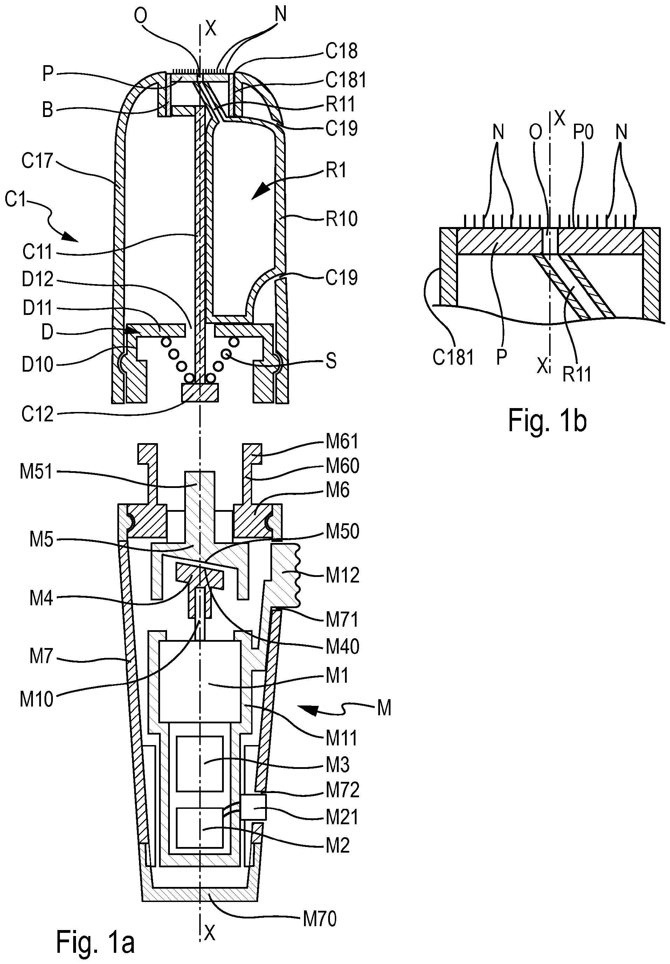

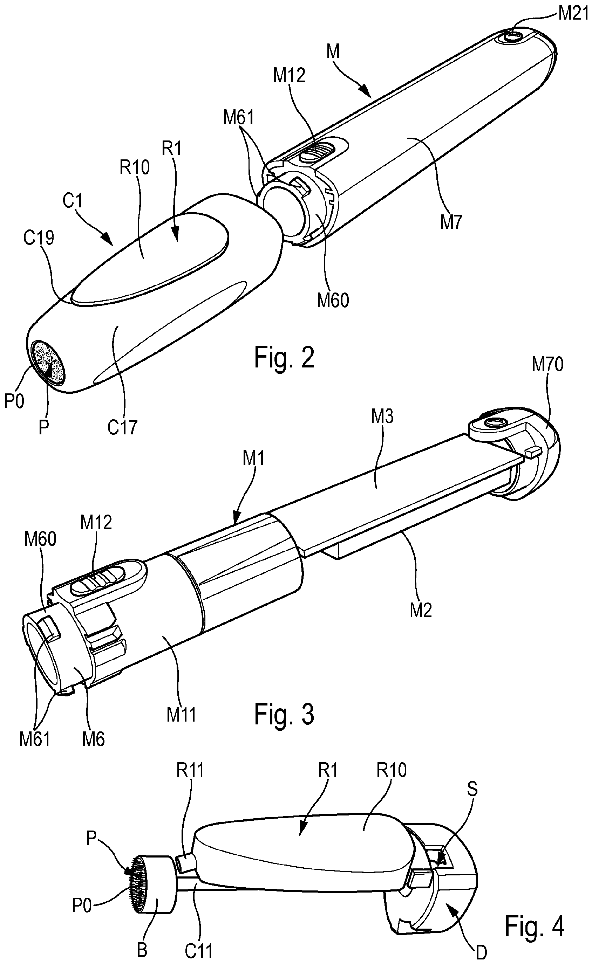

On se référera d'abord aux figures 1a, 1b, 2, 3 et 4 pour décrire en détail le premier mode de réalisation de l'invention. L'applicateur de l'invention comprend deux modules distincts, à savoir un premier module M et un second module C1, qui sont connectables et déconnectables de manière simple et rapide, par l'utilisateur lui-même, par exemple à l'aide de ses deux mains en imprimant un couple et/ou une poussée/traction entre les deux modules. Lorsque les deux modules sont assemblés, l'applicateur présente une configuration générale en forme de stylo avec un axe longitudinal X. L'applicateur peut d'ailleurs être saisi de la même manière qu'un stylo, maintenu entre le pouce et le majeur avec l'index reposant sur l'applicateur.

Le premier module M comprend une coque externe M7 de forme sensiblement cylindrique ou légèrement tronconique. Sa section transversale peut être de forme sensiblement triangulaire. La coque M7 est ouverte à son extrémité supérieure et fermée à son extrémité inférieure par un fond rapporté M70. La coque M7 peut comprendre deux fenêtres latérales M71 et M72 pour la réception de deux boutons de commande M71 et M21, comme on le verra ci-après.

Le premier module M renferme un moteur M1, qui est de préférence un moteur électrique. Il peut s'agir d'un petit moteur rotatif qui entraine un arbre M10 en rotation sur lui-même. On peut aussi prévoir un moteur à électroaimant, un moteur linéaire ou un piézoélectrique. Le moteur M1 est alimenté par une batterie M2 et commandé par une électronique M3, qui va gérer la vitesse de rotation de l'arbre M10, les séquences et les durées d'activation du moteur, etc. Un bouton d'activation externe M21, accessible à travers la fenêtre latérale M71 située à proximité du fond M70, permet à l'utilisateur de mettre l'applicateur sous tension. L'extrémité libre de l'arbre M10 est coiffé d'un capuchon vacillant M4, qui forme une surface de came inclinée M40. La rotation de l'arbre M10 entraine cette surface de came inclinée en rotation sur elle-même, décrivant ainsi un mouvement vacillant rotatif.

Avantageusement, le moteur M1, la batterie M2, l'électronique M3 et le capuchon vacillant M4 sont montés sur un chariot mobile M11 selon l'axe longitudinal X. Le chariot mobile M11 comprend un curseur de sélection M12, accessible à travers la fenêtre latérale M72, qui permet à l'utilisateur de régler ou d'ajuster la profondeur de pénétration des micro-aiguilles, comme on le verra ci-après. Le chariot mobile est toutefois optionnel.

Le premier module M comprend également un piston M4 qui se déplace axialement en va-et-vient. Le piston M4 forme une face inférieure inclinée M50 qui est prise avec la surface de came inclinée M40 du capuchon vacillant M4. Le piston M5 est bloqué en rotation, de sorte que la face inférieure inclinée M50 reste statique, alors que la surface de came inclinée M40 est entrainée en rotation par le moteur M1. Il s'ensuit que le piston M5 est entrainé en déplacement axial, du fait du contact vacillant entre la surface de came M40 et la face inclinée M50. Le piston M5 comprend une tête de transmission M51, située à l'opposé de la face inclinée M50, qui se déplace donc axialement en va-et-vient, lorsque le moteur M1 est activé.

Le premier module M comprend aussi un embout de connexion M6 qui forme une douille de connexion M60 pourvue de profils de connexion M61. L'embout de connexion M6 est monté fixement dans la coque M7, par exemple par encliquetage. L'embout de connexion M6 est traversé par le piston M5, dont la tête de transmission M51 s'étend dans la douille de connexion M60. Le piston M5 peut être guidé axialement dans l'embout de connexion M6, de manière à le bloquer en rotation. On peut ainsi dire que le piston M5 coulisse axialement en va-et-vient dans l'embout de connexion M6. La course axiale du piston M5 est toutefois limitée vers le bas par le capuchon vacillant M4 et vers le haut par l'embout de connexion M6. Le piston M5 est donc coincé entre le capuchon M4 et l'embout M6 avec un degré de liberté purement axial, imposé par le capuchon vacillant M4. La course du piston peut être augmentée ou réduite en agissant sur le curseur de sélection M12, qui déplace le capuchon vacillant M4 par rapport à l'embout de connexion M6, qui reste fixe.

On comprend que ce premier module M contient des organes et composants coûteux, qui ont vocation à être conservés sur une durée prolongée.

Le second module C1 de ce premier mode de réalisation comprend une enveloppe externe C17 de forme sensiblement cylindrique ou légèrement tronconique. Sa section transversale peut être de forme sensiblement triangulaire, complémentaire à celle de la coque M7 du premier module M. L'enveloppe C17 forme une fenêtre centrale C18 à son extrémité supérieure et est ouverte à son extrémité inférieure. La fenêtre centrale C18 peut former un cylindre de guidage axial 181. L'enveloppe C17 comprend une fenêtre latérale C19 pour la réception de l'organe d'actionnement R10 du réservoir R, comme on le verra ci-après.

Le second module C1 comprend une platine P formant une face externe d'application PO sur laquelle sont disposées des micro-aiguilles N. Leur nombre peut varier de 5 à 100. Leur épaisseur peut varier de 0,05 à 0,5 mm. Leur longueur peut varier de 0,1 à 0,7 mm. Ces valeurs sont données à titre purement indicatif. La platine P est traversée par une sortie de produit fluide O, qui peut être centrale ou décalée. On peut aussi prévoir plusieurs sorties de produit fluide, par exemple disposées en cercle. La sortie de produit fluide peut aussi se faire en périphérie de la platine P. Eventuellement, les sorties peuvent se faire à travers des micro-aiguilles creuses. A vrai dire, la conception de la face d'application PO n'est pas critique pour l'invention, dans la mesure où elle présente des micro-aiguilles N et permet la distribution de produit fluide.

La platine P est montée sur ou dans une bague de support B à laquelle est fixée une tige de transmission C11, qui s'étend axialement de manière centrée. La tige de transmission C11 comprend un talon de contact C12 destiné à venir en contact appuyé avec la tête de transmission M51 du premier module M.

Le second module C1 comprend aussi un manchon de connexion D qui est monté fixement dans l'ouverture inférieure de l'enveloppe externe C17, par exemple par encliquetage. Ce manchon D comprend un logement de connexion D10 adapté à recevoir les profils de connexion M61 de la douille de connexion M60. Le manchon D comprend également une bride annulaire D11 qui forme un passage central D12, à travers lequel passe la tige de transmission C11. Le talon de contact C12 est disposé dans le manchon D, avec un ressort de rappel S prenant appui entre le talon C11 et la bride D11, de sorte que la platine P est sollicitée vers l'intérieur de la fenêtre C18.

Le second module C1 comprend également un réservoir de produit fluide cosmétique R1 qui est relié à la sortie de produit fluide O par un conduit d'alimentation R11. Le réservoir R1 comprend une paroi d'actionnement R10 qui est accessible à travers la fenêtre latérale C19 de l'enveloppe C17. La paroi d'actionnement R10 est déformable, élastiquement ou définitivement. Une pression exercée par un doigt de l'utilisateur, généralement l'index, a pour effet de réduire le volume utile du réservoir R1 et de mettre son contenu sous pression, ce qui conduit à refouler une partie de son contenu à travers le conduit d'alimentation R11 qui débouche au niveau de la sortie de produit fluide O. une dose de produit fluide est ainsi distribué sur la face d'application PO.

Le réservoir R1 peut être réalisé de manière monobloc avec une matière plastique appropriée, comme un élastomère. Sa paroi d'actionnement R10 peut présenter une épaisseur de paroi réduite pour lui conférer une plus grande élasticité. Le réservoir R1 est logé à côté de la tige de transmission C11, sur le côté droit sur la figure 1a. En variante, le réservoir R1 peut entourer partiellement ou complètement la tige de transmission C11. On pourrait prévoir deux parois d'actionnement opposées, que l'utilisateur pourrait enfoncer entre le pouce et l'index ou le majeur (variante non revendiquée).

Le second module C1, de par sa simplicité de conception et sa passivité (pas de composant électrique ou électronique), constitue une entité peu coûteuse, qui peut être considérée comme une cartouche ou une recharge remplaçable. L'applicateur peut par exemple être commercialisé sous la forme d'un kit comprenant un premier module M et plusieurs seconds modules C11. Une protection pourrait être recherchée pour un tel kit.

En terme de fonction, on peut dire que le second module C1 comprend un organe d'actionnement R10, sous la forme de la paroi souple du réservoir R1, qui permet de distribuer du produit fluide au niveau de la face d'application PO. Cet organe d'actionnement agit directement sur le réservoir R1, sans aucun intermédiaire. Il est accessible lorsque les deux modules sont connectés l'un à l'autre. L'utilisateur peut l'actionner manuellement à l'aide d'un ou de plusieurs doigts.

Pour connecter le second module C1 au premier module M, il suffit d'engager axialement la douille de connexion M60 dans le manchon de connexion D. Ce faisant, la tête de transmission M51 du piston M5 va venir en contact avec le talon de contact C12 de la tige de transmission C11. Le ressort S sera éventuellement comprimé. Lorsque les profils de connexion M61 seront en butée contre la bride annulaire D11, l'utilisateur pourra effectuer un mouvement de rotatif à l'un des modules pour verrouiller les profils de connexion M61 dans le logement de connexion D10 du manchon D. Un léger « clic » tactile et/ou sonore pourra indiquer que la connexion est accomplie.

L'utilisateur peut alors sélectionner la profondeur de pénétration des micro-aiguilles en agissant sur le curseur M12. Il peut appuyer sur la paroi d'actionnement R10 pour distribuer une dose de produit fluide cosmétique au niveau de la face d'application PO. Un appui sur le bouton d'activation M21 met le moteur en action, ce qui a pour effet de faire tourner le capuchon vacillant M4, qui va déplacer le piston M5 en va-et-vient axial, qui va appuyer sur le talon C12 à l'encontre du ressort S. La tige C11 transmet la vibration à la platine P avec ses micro-aiguilles N, qui vont percer la couche superficielle de la peau de l'utilisateur, qui est déjà enduite de produit fluide cosmétique. Le produit fluide pénètre ainsi superficiellement la peau pour une action renforcée.

On peut avantageusement prévoir au niveau de l'électronique M3 un arrêt du moteur M1, lorsque le second module C1 n'est pas connecté au premier module M.

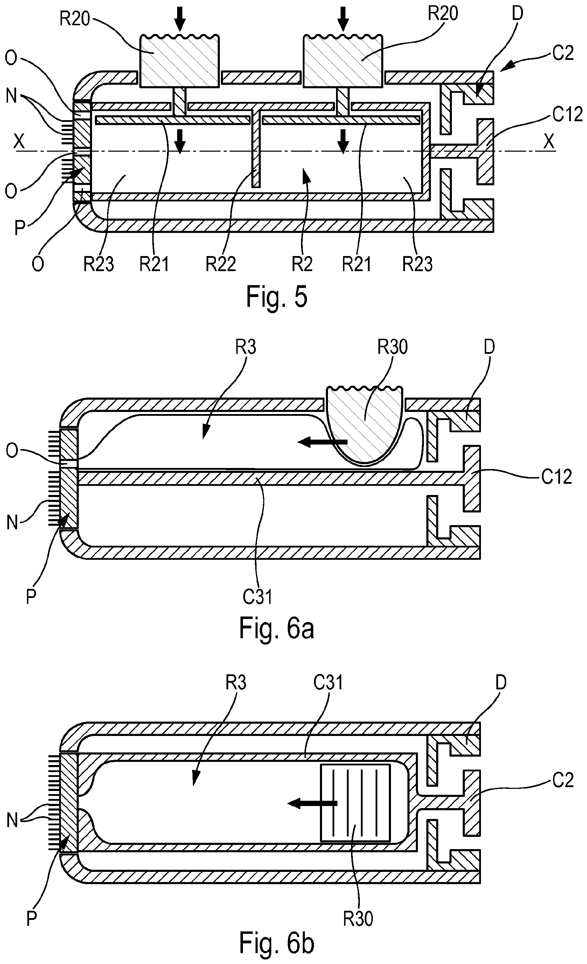

On se référera maintenant aux figures 5 à 9 pour décrire plusieurs modes de réalisation pour le second module de l'applicateur, particulièrement au niveau du réservoir de produit fluide, de son organe d'actionnement et de la transmission de la vibration entre le talon de contact et la platine. Le but de ces modes de réalisation est de montrer que le réservoir peut être actionné de diverses manières et même remplir une fonction de transmission. Les vibrations peuvent également servir à distribuer le produit fluide du réservoir. Les seconds modules des figures 5 à 9 sont adaptés à être raccordés au premier module M du premier mode de réalisation.

Ainsi, sur la figure 5, on voit un second module C2 avec une platine P et un manchon D comparable ou identique à celui du premier mode de réalisation. En revanche, le réservoir R2 est différent, puisqu'il est rigide, mais comprenant deux compartiments R23 séparée par une cloison R22, qui permet quand même une communication entre eux. Ce module C2 comprend deux poussoirs R20 connectés respectivement à deux pistons R21. Par appui sur un poussoir R20, une dose de produit fluide est refoulée à travers la platine P. La dose correspond environ à la moitié de la contenance du réservoir. Bien entendu, on peut ne prévoir qu'un seul poussoir R20, ou au contraire plus de deux. Il faut aussi remarquer que c'est le réservoir R2 qui remplit la fonction de la tige de transmission C11 du premier mode de réalisation en transmettant à la platine P les vibrations reçues par le talon de contact C12. En variante, le réservoir compartimenté peut être formée par une poche souple que l'on écrase avec les pistons R21.

Sur les figures 6a et 6b, on peut voir un autre second module C3, dans lequel le réservoir R3 comprend une poche souple écrasable au moyen d'un curseur coulissant R30. La poche souple repose sur une plaque de transmission C31, qui remplit la même fonction que la tige de transmission C11 du premier mode de réalisation. Le déplacement du curseur coulissant R30 peut être parfaitement linéaire ou au contraire saccadé par des crans pour délimiter des doses de produit fluide.

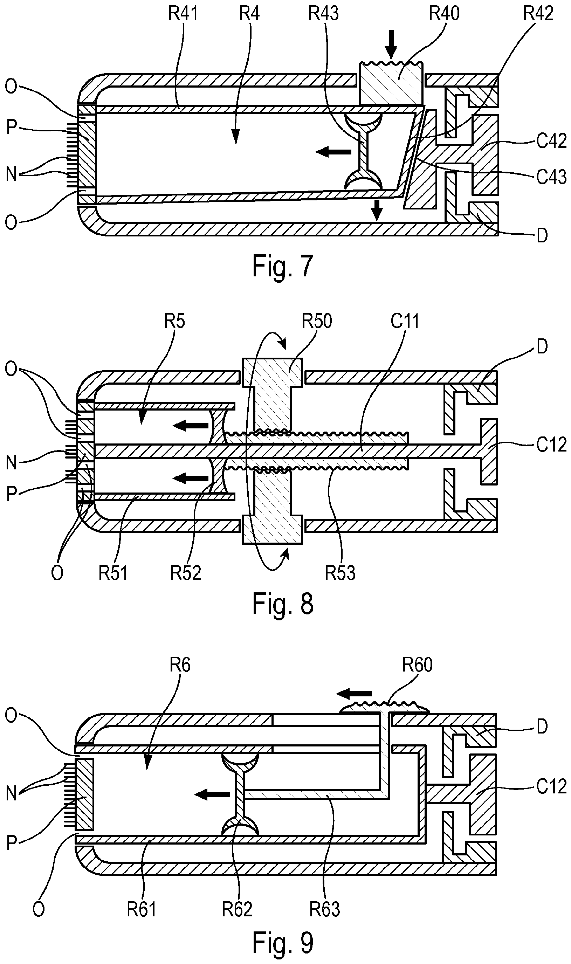

Sur la figure 7, on peut voir un autre second module C4, dans lequel le réservoir R4 comprend un fût de coulissement R41, qui est monté de manière à pouvoir pivoter légèrement en étant connecté à la platine P. Le fût R41 comprend un fond en biseau R42 qui peut sélectivement venir en prise avec une surface biseautée C43 du talon de contact C42. Un poussoir R40 permet de faire pivoter le fût R41 pour amener le fond R41 en contact de la surface biseautée C43 et ainsi établir une continuité de transmission. Le fût contient un piston pousseur R43 qui se déplace dans le fût R41 en direction de la platine P avec les vibrations générées par le premier module M. Le réservoir R4 remplit ainsi une fonction de transmission de vibration embrayable/débrayable par appui sur le poussoir.

Sur la figure 8, on peut voir un autre second module C5, dans lequel le réservoir R5 comprend un fût de coulissement R51, qui remplit également une fonction de transmission de vibration. Le fût R51 contient un piston pousseur R52 qui est déplaçable dans le fût au moyen d'une tige filetée R53 qui est en prise avec une bague d'actionnement rotative R50. Le déplacement de la bague rotative R50 peut être linéaire ou cranté.

En variante, la bague rotative peut agir sur le piston pousseur du réservoir par l'intermédiaire d'une crémaillère. On peut également imaginer que bague rotative R50 serve à tordre une poche souple, qui peut s'étendre à côté ou autour de la tige de transmission C11.

Sur la figure 9, on peut voir un autre second module C6, dans lequel le réservoir R6 comprend un fût de coulissement R61, qui remplit également une fonction de transmission de vibration. Le fût R61 contient un piston pousseur R62 qui est déplaçable dans le fût au moyen d'un curseur coulissant R60 relié au piston pousseur R62 par une tringle R63.

A travers les exemples des figures 5 à 9, ainsi que du premier mode de réalisation, on comprend que le réservoir R1, R2, R3, R4, R5, R6 peut remplir différentes fonctions, en plus de sa fonction de base. Il peut transmettre les vibrations aux micro-aiguilles, servir d'embrayage, de variateur de la transmission ou encore de variateur de la pénétration des micro-aiguilles.

Dans tous les modes de réalisation, le second module C1, C2, C3, C4, C5, C6 comprend un organe d'actionnement R10, R20, R30, R40, R50, R60 qui est accessible et manipulable à la main, même lorsque le second module est raccordé au premier module. On a vu que l'organe d'actionnement peut se présenter sous différentes formes (poussoir, curseur coulissant, bague rotative) et se déplace soit perpendiculairement à l'axe longitudinal X, soit axialement, soit en rotation. Il peut y avoir plusieurs organes d'actionnement (R20) pour délimiter des doses. Le déplacement de l'organe d'actionnement peut aussi être cranté. L'organe d'actionnement peut faire partie intégrante du réservoir ou constituer un organe associé au réservoir.

On peut également noter que la sortie de produit fluide O peut être centrale, décalée, disposée en cercle unique ou concentrique. Sur la figure 9, on peut voir que la sortie de produit fluide O s'étend tout autour de la platine P qui supporte les micro-aiguilles N. cette platine est montée dans le fût de coulissement R61.

Grâce à l'invention, on dispose d'un applicateur de produit cosmétique à micro-aiguilles vibrantes qui est conçu en deux modules séparables, à savoir un module « moteur » et un module « réservoir/micro-aiguilles » que l'on peut considérer comme une cartouche ou recharge, dont la conception est simple, ce qui le rend peu coûteuse et donc jetable. La physionomie de l'applicateur comparable à un stylo rend aussi sa manipulation intuitive.

## Revendications

1. Applicateur à micro-aiguilles pour appliquer un produit fluide sur la peau et le faire pénétrer dans la peau, l'applicateur définissant un axe longitudinal X et comprenant :
- une face d'application (PO) pourvue d'au moins une sortie de produit fluide (O) et de plusieurs micro-aiguilles (N),
- un moteur (M1) pour faire vibrer les micro-aiguilles (N),
- un réservoir de produit fluide (R1 ; R2 ; R3 ; R4 ; R5 ; R6 ; R7) relié à la sortie de produit fluide (O),
- un organe d'actionnement manuel (R10 ; R20 ; R30 ; R40 ; R50 ; R60 ; R70) qui agit directement sur le volume du réservoir pour acheminer le produit fluide du réservoir de produit fluide (R1 ; R2 ; R3 ; R4 ; R5 ; R6 ; R7) à la sortie de produit fluide (O), l'utilisateur agissant à l'aide d'un doigt sur l'organe d'actionnement manuel (R10 ; R20 ; R30 ; R40 ; R50 ; R60 ; R70),
**caractérisé en ce qu'**il comprend deux modules distincts (M, C1 ; C2 ; C3 ; C4 ; C5 ; C6 ; C7) raccordés axialement l'un à l'autre de manière amovible, à savoir :
- un premier module (M) abritant le moteur (M1) et des accessoires pour faire fonctionner le moteur (M), et
- un second module (C1 ; C2 ; C3 ; C4 ; C5 ; C6 ; C7) abritant le réservoir de produit fluide (R1 ; R2 ; R3 ; R4 ; R5 ; R6 ; R7) et formant la face d'application (PO) et l'organe d'actionnement manuel (R10 ; R20 ; R30 ; R40 ; R50 ; R60 ; R70), l'organe d'actionnement manuel (R10 ; R20 ; R30 ; R40 ; R50 ; R60 ; R70) étant accessible et manipulable manuellement, lorsque les deux modules (M, C1 ; C2 ; C3 ; C4 ; C5 ; C6 ; C7) sont connectés l'un à l'autre.

2. Applicateur selon la revendication 1, dans lequel le réservoir de produit fluide (R1 ; R2 ; R3 ; R4 ; R5 ; R6 ; R7) est à volume variable, le déplacement de l'organe d'actionnement manuel (R10; R20 ; R30 ; R40 ; R50 ; R60 ; R70) engendrant une diminution de volume du réservoir de produit fluide (R1 ; R2 ; R3 ; R4 ; R5 ; R6 ; R7), de sorte qu'une partie de son contenu est refoulée vers la sortie de produit fluide (O).

3. Applicateur selon la revendication 1 ou 2, dans lequel l'organe d'actionnement manuel (R10 ; R20 ; R30 ; R40 ; R50 ; R60 ; R70) est un poussoir latéral manuel.

4. Applicateur selon la revendication 3, dans lequel le poussoir latéral manuel est choisi parmi :
- un ou plusieurs poussoir(s) (R10 ; R20 ; R40 ; R50) à déplacement transversal par rapport à l'axe longitudinal X,
- un curseur coulissant (R30 ; R70) parallèlement à l'axe longitudinal X, et
- une bague rotative (R60) qui tourne autour de l'axe longitudinal X.

5. Applicateur selon l'une quelconque des revendications précédentes, dans lequel le second module (C1 ; C2 ; C3 ; C4 ; C5 ; C6 ; C7) comprend une extrémité de connexion pourvue de moyens de couplage (C12 ; C42) au premier module (M) pour transmettre les vibrations générées par le moteur (M1) et des moyens de transmission (C11 ; C31 ; R2 ; R4 ; R5 ; R6) pour transmettre aux micro-aiguilles (N) les vibrations reçus par les moyens de couplage (C12 ; C42).

6. Applicateur selon la revendication 5, dans lequel les moyens de transmission sont formés par le réservoir de produit fluide (R2 ; R4 ; R5 ; R6).

7. Applicateur selon la revendication 6, dans lequel le réservoir de produit fluide (R4 ; R5) est débrayable des moyens de couplage (C42 ; C12) entre une position de repos débrayée et une position active embrayée, l'organe d'actionnement (R40 ; R50) amenant le réservoir de produit fluide (R4 ; R5) en position embrayée.

8. Applicateur selon la revendication 5, 6 ou 7, dans lequel les moyens de transmission comprenant une tige de transmission (C11), le réservoir de produit fluide (R1) s'étendant à côté de la tige de transmission (C11).

9. Applicateur selon la revendication 5, 6 ou 7, dans lequel les moyens de transmission comprenant une plaque de transmission (C31), le réservoir de produit fluide (R3) comprenant une poche souple reposant sur la plaque de transmission (C31), l'organe d'actionnement (R30) écrasant la poche souple sur la plaque de transmission (C31).

10. Applicateur selon l'une quelconque des revendications précédentes, dans lequel le réservoir (R1), l'organe d'actionnement (R10) et la sortie de produit fluide (R11) sont réalisés de manière monobloc, avantageusement avec un élastomère.

11. Applicateur selon l'une quelconque des revendications précédentes, présentant une configuration générale de stylo, adapté à être saisi entre le pouce et le majeur avec l'organe d'actionnement manuel (R10 ; R20 ; R30 ; R40 ; R50 ; R60 ; R70) actionnable au moyen de l'index, le premier module (M), et avantageusement aussi le second module (C1 ; C2 ; C3 ; C4 ; C5 ; C6 ; C7), présentant une section transversale sensiblement triangulaire.

12. Applicateur selon l'une quelconque des revendications précédentes, dans lequel le second module (C1 ; C2 ; C3 ; C4 ; C5 ; C6 ; C7) est raccordé de manière amovible au premier module () par une connexion combinant un mouvement axial et un mouvement rotatif.

13. Applicateur selon l'une quelconque des revendications précédentes, dans lequel la face d'application (PO) comprend une platine (P) dotée de micro-aiguille (N) et traversée par la sortie de produit fluide (O).

14. Applicateur selon l'une quelconque des revendications précédentes, comprenant en outre des moyens d'ajustement de la profondeur de pénétration des micro-aiguilles (N), agissant avantageusement sur la position axiale du moteur (M1) dans le premier module (M).

15. Applicateur selon l'une quelconque des revendications précédentes, comprenant en outre des moyens de commande (M3) du moteur (M1), qui le désactivent automatiquement lorsque les deux modules (M, C1 ; C2 ; C3 ; C4 ; C5 ; C6 ; C7) ne sont pas connectés.

## Patentansprüche

1. Applikator mit Mikronadeln zum Auftragen eines Fluidprodukts auf die Haut und zum Eindringen des Fluidprodukts in die Haut, wobei der Applikator eine Längsachse X definiert und umfasst:
- eine Applikationsfläche (PO), die mit zumindest einem Fluidproduktauslass (O) und mit mehreren Mikronadeln (N) versehen ist,
- einen Motor (M1), um die Mikronadeln (N) in Schwingung zu versetzen,
- ein Fluidproduktreservoir (R1; R2; R3; R4; R5; R6; R7), das mit dem Fluidproduktauslass (O) verbunden ist,
- ein manuelles Betätigungsorgan (R10; R20; R30; R40; R50; R60; R70), das direkt auf das Volumen des Reservoirs einwirkt, um das Fluidprodukt aus dem Fluidproduktreservoir (R1; R2; R3; R4; R5; R6; R7) zum Fluidproduktauslass (O) zu leiten, wobei der Benutzer mit Hilfe eines Fingers auf das manuelle Betätigungsorgan (R10; R20; R30; R40; R50; R60; R70) einwirkt,
**dadurch gekennzeichnet, dass** er zwei separate Module (M, C1; C2; C3; C4; C5; C6; C7) umfasst, die axial lösbar miteinander verbunden sind, nämlich:
- ein erstes Modul (M), das den Motor (M1) und Zubehörteile zum Betreiben des Motors (M) aufnimmt, und
- ein zweites Modul (C1; C2; C3; C4; C5; C6; C7), das das Fluidproduktreservoir (R1; R2; R3; R4; R5; R6; R7) aufnimmt und die Applikationsfläche (PO) und das manuelle Betätigungsorgan (R10; R20; R30; R40; R50 ; R60; R70) bildet, wobei das manuelle Betätigungsorgan (R10; R20; R30; R40; R50; R60; R70) dann zugänglich und manuell bedienbar ist, wenn die beiden Module (M, C1; C2; C3; C4; C5; C6; C7) miteinander verbunden sind.

2. Applikator nach Anspruch 1,
wobei das Fluidproduktreservoir (R1; R2; R3; R4; R5; R6; R7) ein variables Volumen hat, wobei die Verlagerung des manuellen Betätigungsorgans (R10; R20; R30; R40; R50; R60; R70) eine Volumenverringerung des Fluidproduktreservoirs (R1; R2; R3; R4; R5; R6; R7) bewirkt, so dass ein Teil seines Inhalts zum Fluidproduktauslass (O) hin verdrängt wird.

3. Applikator nach Anspruch 1 oder 2,
wobei das manuelle Betätigungsorgan (R10; R20; R30; R40; R50; R60; R70) ein seitlicher Handschieber ist.

4. Applikator nach Anspruch 3,
wobei der seitliche Handschieber ausgewählt ist aus:
- einem oder mehreren Schieber(n) (R10; R20; R40; R50), der/die quer zur Längsachse X verschiebbar ist/sind,
- einem Schieber (R30; R70), der parallel zur Längsachse X gleitbeweglich ist, und
- einem Drehring (R60), der sich um die Längsachse X dreht.

5. Applikator nach einem der vorhergehenden Ansprüche,
wobei das zweite Modul (C1; C2; C3; C4; C5; C6; C7) ein Verbindungsende umfasst, das mit Kopplungsmitteln (C12; C42) zum Koppeln mit dem ersten Modul (M) versehen ist, um die vom Motor (M1) erzeugten Schwingungen zu übertragen, sowie mit Übertragungsmitteln (C11; C31; R2; R4; R5; R6), um die von den Kopplungsmitteln (C12; C42) aufgenommenen Schwingungen auf die Mikronadeln (N) zu übertragen.

6. Applikator nach Anspruch 5,
wobei die Übertragungsmittel aus dem Fluidproduktreservoir (R2; R4; R5; R6) gebildet sind.

7. Applikator nach Anspruch 6,
wobei das Fluidproduktreservoir (R4; R5) zwischen einer ausgerückten Ruhestellung und einer eingerückten Wirkstellung von den Kopplungsmitteln (C42; C12) ausgerückt werden kann, wobei das Betätigungsorgan (R40; R50) das Fluidproduktreservoir (R4; R5) in die eingerückte Stellung bringt.

8. Applikator nach Anspruch 5, 6 oder 7,
wobei die Übertragungsmittel eine Übertragungsstange (C11) umfassen, wobei das Fluidproduktreservoir (R1) neben der Übertragungsstange (C11) verläuft.

9. Applikator nach Anspruch 5, 6 oder 7,
wobei die Übertragungsmittel eine Übertragungsplatte (C31) umfassen, das Fluidproduktreservoir (R3) einen nachgiebigen Beutel umfasst, der auf der Übertragungsplatte (C31) aufliegt, und das Betätigungsorgan (R30) den nachgiebigen Beutel auf der Übertragungsplatte (C31) zusammendrückt.

10. Applikator nach einem der vorhergehenden Ansprüche,
wobei das Reservoir (R1), das Betätigungsorgan (R10) und der Fluidproduktauslass (R11) einstückig ausgebildet sind, vorteilhafterweise mit einem Elastomer.

11. Applikator nach einem der vorhergehenden Ansprüche,
wobei er in Gestalt eines Schreibstifts vorliegt, der zwischen Daumen und Mittelfinger ergriffen werden kann, wobei das manuelle Betätigungsorgan (R10; R20; R30; R40; R50; R60; R70) mit dem Zeigefinger betätigt werden kann, wobei das erste Modul (M) und vorteilhafterweise auch das zweite Modul (C1; C2; C3; C4; C5; C6; C7) einen im Wesentlichen dreieckigen Querschnitt aufweist.

12. Applikator nach einem der vorhergehenden Ansprüche,
wobei das zweite Modul (C1; C2; C3; C4; C5; C6; C7) durch eine Verbindung, die eine axiale Bewegung und eine Drehbewegung kombiniert, mit dem ersten Modul lösbar verbunden ist.

13. Applikator nach einem der vorhergehenden Ansprüche,
wobei die Applikationsfläche (PO) eine Platine (P) umfasst, die mit Mikronadeln (N) versehen ist und von dem Fluidproduktauslass (O) durchquert wird.

14. Applikator nach einem der vorhergehenden Ansprüche,
ferner umfassend Mittel zum Einstellen der Eindringtiefe der Mikronadeln (N), die vorteilhafterweise auf die axiale Stellung des Motors (M1) im ersten Modul (M) einwirken.

15. Applikator nach einem der vorhergehenden Ansprüche,
ferner umfassend Steuermittel (M3) zum Steuern des Motors (M1), die diesen automatisch abschalten, wenn die beiden Module (M, C1; C2; C3; C4; C5; C6; C7) nicht verbunden sind.

## Claims

1. A microneedle applicator for applying a fluid product on the skin and for causing it penetrate into the skin, the applicator defining a longitudinal axis X and comprising:
- an application face (PO) provided with at least a fluid product outlet (O) and a plurality of microneedles (N),
- a motor (M1) for causing the microneedles (N) to vibrate,
- a fluid product reservoir (R1; R2; R3; R4; R5; R6; R7) connected to the fluid product outlet (O),
- a manual actuating member (R10; R20; R30; R40; R50; R60; R70) which acts directly on the volume of the reservoir for conveying the fluid product from the fluid product reservoir (R1; R2; R3; R4; R5; R6; R7) to the fluid product outlet (O), the user taking action by means of a finger on the manual actuating member (R10; R20; R30; R40; R50; R60; R70), **characterised in that** it comprises two distinct modules (M, C1; C2; C3, C5; C6; C7) removably axially connected to one another, namely:
- a first module (M) housing the motor (M1) and accessories for operating the motor (M), and
- a second module (C1; C2; C3; C4; C5; C6; C7) housing the fluid product reservoir (R1; R2; R3; R4; R5; R6; R7) and forming the application face (PO) and the manual actuating member (R10; R20; R30; R40; R50; R60; R70), the manual actuating member (R10; R20; R30; R40; R50; R60; R70) being manually accessible and manipulable, when the two modules (M, C1; C2; C3; C4; C5; C6; C7) are connected to each other.

2. The applicator according to Claim 1, wherein the fluid product reservoir (R1; R2; R3; R4; R5; R6; R7) has a variable volume, the movement of the manual actuating member (R10 ; R20; R30; R40; R50; R60; R70) leading to a decrease in volume of the fluid product reservoir (R1; R2; R3; R4; R5; R6; R7), such that some of its content is repelled to the fluid product outlet (O).

3. The applicator according to Claim 1 or 2, wherein the manual actuating member (R10; R20; R30; R40; R50; R60; R70) is a manual lateral pusher.

4. The applicator according to Claim 3, wherein the manual lateral pusher is selected from:
- one or a plurality of pushers (R10; R20; R40; R50) with transverse movement with respect to the longitudinal axis X;
- a slider (R30; R70) parallel to the longitudinal axis X, and
- a rotary ring (R60) which rotates around the longitudinal axis X.

5. The applicator according to any one of the preceding claims, wherein the second module (C1; C2; C3; C4; C5; C6; C7) comprises a connection end provided with coupling means (C12; C42) to the first module (M) for transmitting the vibrations generated by the motor (M1) and transmission means (C11; C31; R2; R4; R5; R6) for transmitting to the microneedles (N) the vibrations received by the coupling means (C12; C42).

6. The applicator according to Claim 5, wherein the transmission means are formed by the fluid product reservoir (R2; R4; R5; R6).

7. The applicator according to Claim 6, wherein the fluid product reservoir (R4; R5) can be disengaged from the coupling means (C42; C12) between a disengaged rest position and an engaged active position, the actuating member (R40; R50) moving the fluid product reservoir (R4; R5) into the engaged position.

8. The applicator according to Claim 5, 6 or 7, wherein the transmission means comprises a transmission rod (C11), the fluid product reservoir (R1) extending beside the transmission rod (C11).

9. The applicator according to Claim 5, 6 or 7, wherein the transmission means comprises a transmission plate (C31), the fluid product reservoir (R3) comprising a flexible pouch resting on the transmission plate (C31), the actuating member (R30) squeezing the flexible pouch onto the transmission plate (C31).

10. The applicator according to any one of the preceding claims, wherein the reservoir (R1), the actuating member (R10) and the fluid product outlet (R11) are made as a single part, advantageously with an elastomer.

11. The applicator according to any one of the preceding claims, having a general pen-shaped configuration, adapted to be held between the thumb and middle finger with the manual actuating member (R10; R20; R30; R40; R50; R60; R70) operable by means of the index finger, the first module (M), and advantageously also the second module (C1; C2; C3; C4; C5; C6; C7), having a substantially triangular cross-section.

12. The applicator according to any one of the preceding claims, wherein the second module (C1; C2; C3; C4; C5; C6; C7) is removably connected to the first module (M) by a connection combining axial and rotary motions.

13. The applicator according to any one of the preceding claims, wherein the application face (PO) may comprise a plate (P) equipped with microneedles (N), and passed through by the fluid product outlet (O).

14. The applicator according to any one of the preceding claims, further comprising means for adjusting the penetration depth of the microneedles (N), acting advantageously on the axial position of the motor (M1) in the first module (M).

15. The applicator according to any one of the preceding claims, further comprising control means (M3) of the motor (M1), which automatically deactivate it when the two modules (M, C1; C2; C3; C4; C5; C6; C7) are not connected.
